# EUROPEAN PATENT APPLICATION

(11) **EP 3 616 705 A2**
(43) Date of publication of application: **04.03.2020**
(21) Application number: 18841028.6
(22) Date of filing: 31.07.2018
(51) Int. Cl.: A61K 36/81, A23L 33/105

(54) **COMPOSITION FOR IMPROVING PREVENTION AND TREATMENT OF FATTY LIVER CONTAININGAMOMUM VILOSUM**

(30) Priority: 03.08.2017 KR 20170098490
(71) Applicant: Corporation Ilwonbio, Iksan-si, Jeollabuk-do 54576 (KR)
(72) Inventor: KWON, Kang Beom, Jeonju-si Jeollabuk-do 54823 (KR); KIM, Ha Rim, Jeonju-si Jeollabuk-do 54866 (KR); LEE, Guem San, Jeollabuk-do 55342 (KR)
(74) Representative: Gulde & Partner
(86) International application number: PCT/KR2018/008718
(87) International publication number: WO 2019/027244

(57) **Abstract**

The present invention relates to a composition for preventing, treating, or alleviating fatty liver including an *Amomum villosum* extract as an active ingredient.

The present invention relates to a composition for preventing, treating, or alleviating fatty liver, wherein the *Amomum villosum* extra is obtained through extraction with water and included in a dose of 100 mg/kg to 500 mg/kg based on oral administration to a mouse.

The present invention provides a composition for preventing, treating, or alleviating fatty liver including an *Amomum villosum* extract as an active ingredient, wherein the inhibitory efficacy and mechanism of the *Amomum villosum* extract on fatty liver production are confirmed *in vitro* and *in vivo* through the inhibition of intrahepatic TG, the inhibition of ER stress-related gene expression, and the inhibition of fat synthesis-related gene expression.

## Description

### [Technical Field]

The present invention relates to a composition for preventing or treating fatty liver, which includes an *Amomum villosum* extract as an active ingredient.

The present invention provides a composition for preventing, treating, or alleviating fatty liver, which includes an *Amomum villosum* extract as an active ingredient, wherein the *Amomum villosum* extract is obtained through extraction with water, and the inhibitory efficacy and mechanism of the *Amomum villosum* extract on fatty liver production are confirmed *in vitro* and *in vivo* through the inhibition of intrahepatic TG, the inhibition of ER stress-related gene expression, and the inhibition of fat synthesis-related gene expression.

### [Background Art]

Generally, the percentage of fat in a healthy liver is 5%, and liver where more fat than this is accumulated is referred to as fatty liver. Recently, as nutritional conditions have improved and adult diseases have increased, fatty liver patients are on the rise. Fatty liver is a disease that results from intrahepatic fat accumulation and synthesis and abnormal fat metabolism due to excessive fat intake.

Fatty liver can be divided into alcoholic fatty liver caused by heavy drinking and non-alcoholic fatty liver due to obesity, diabetes, hyperlipidemia, drugs, and the like. Alcoholic fatty liver is caused by the ingestion of large amounts of alcohol, which promotes intrahepatic fat synthesis and lacks normal energy metabolism.

In other words, the main causes of fatty liver are drinking and obesity, accompanied by diseases such as hyperlipidemia having high blood fat levels, diabetes, or the like, and drugs such as corticosteroids (steroids), female hormones, or the like may also be causes thereof.

Most cases of fatty liver are asymptomatic and look healthy, showing fatigue and general malaise and sometimes upper right abdominal discomfort or slight pain. Therefore, fatty liver is not clearly detected, but rather, in many cases, abnormal findings are identified mostly in a blood test for checking liver functions or in ultrasound scanning.

Treatment methods for these fatty liver cases vary according to alcoholic fatty liver and non-alcoholic fatty liver.

First, the treatment of alcoholic fatty liver starts with stopping drinking through lifestyle improvement since it is caused by excessive drinking, checkup of health conditions, and continuous management. Second, other than stopping or reducing drinking, antioxidants (vitamin E, vitamins B and C) are used as therapeutic agents. Ursodeoxycholic acid (UDCA), silymarin, or the like, which are hepatocyte protectors, is used to protect the liver.

The treatment methods of non-alcoholic fatty liver include lifestyle improvement, surgical treatment, chemotherapy, and the like.

70% to 80% of patients with fatty liver disease are obese, and many other patients are overweight. Thus, weight loss improves insulin sensitivity, resulting in alleviated fatty liver, and it has recently been reported that, even when only about 5% of body weight is lost, insulin resistance is improved and liver function levels are improved. Therefore, lifestyle improvement is more important than anything.

Second, bariatric surgery was not used much in the past, but as highly obese patients have recently increased, surgery is recommended in the case of morbid obesity (body mass index > 40 kg/m²) or body mass index > 35 kg/m² accompanied by risk factors such as diabetes, hypertension, or the like. Examples thereof include 1) surgery for reducing food intake by inducing early satiety (gastric banding), 2) procedure to bypass the small intestine to thereby induce malabsorption (jejunoileal bypass), and 3) surgery for achieving both effects (Roux-en-Y gastric bypass and biliopancreatic diversion).

Third, for chemotherapy, diabetes therapeutic drugs, insulin sensitizers, antioxidants, hepatocyte protectors, and hyperlipidemia treatment drugs are used. When diabetes therapeutic drugs and insulin sensitizers are used, the level of glucose receptors is increased to promote glucose uptake in muscle and promote differentiation of adipocytes, reducing intrahepatic fat deposition, thereby providing an effect of treating fatty liver and steatohepatitis. In addition, the use of antioxidants is being studied to reduce oxidative stress, which is one of the mechanisms of fatty liver disease, and there is a report of the efficacy of hyperlipidemia treatment drugs on related fatty liver.

However, there are few useful drugs for pharmacological treatment of fatty liver, and exercise and diet are recommended. In addition, it has also been reported that the use of the above drugs causes various side effects, including lactic acidosis, mitochondrial damage to muscles, inflammation, fibrosis, and the like. Fatty liver is a relatively mild disease that can be recovered initially by dietary control, but is a terrible disease in which the liver is unrecoverable due to liver damage, when it persists for a long period of time. Therefore, these fatty liver drugs should be selected in consideration of no occurrence of side effects for short-term administration and even for long-term administration.

Therefore, the applicant of the present invention completed the present invention, which uses an *Amomum villosum* extract, and thus does not cause side effects, does not incur toxicity even upon long-term administration, and provides an effect of preventing, treating, or alleviating fatty liver.

Amomum villosum refers to clumps of ripe fruits or seeds of *Amomum villosum* Loureiro var. *xanthioides* T.L. Wu et Senjen or *Amomum villosum* Loureiro, which belongs to the family Zingiberaceae, and in the present invention, *Amomum villosum* fruits were used as a raw material.

*Amomum villosum* Loureiro is mainly produced in Fujian, Guangdong, Guangxi, and Yunnan, and *Amomum villosum* Loureiro var. *xanthioides* T.L. Wu et Senjen is mainly produced in regions of Indochina, such as Cambodia, India, Laos, Myanmar, Thailand, Vietnam, and the like, and some regions of China, such as Guangxi and Yunnan (Flora of China, v24:347-356.2000). Due to the variety of production places, in ancient times, *Amomum villosum* Loureiro var. *xanthioides* T.L. Wu et Senjen was separately called "*Amomum xanthioides* Wall".

Meanwhile, the fruit of *Amomum longiligulare* T.L. Wu, which is taken as genuine in China but is taken as fake in Korea, is also mass-distributed. However, as a result of conducting research on RP-HPLC, Shen et al. (SHEN Li WANG Yang JIANG Ku et al. Comparison of HPLC Fingerprints of Amomum villosum Lour., Amomurn villosum Lour. var. xanthioides T.L. Wu et Senjen and Amomum longiligulare T.L. Wu. Chinese Pharmaceutical Journal. 2016: 51(12); 1039-1043.) verified that, although three types of *Amomum villosum* exhibited high similarities in terms of ingredients, *Amomum longiligulare* T.L. Wu and *Amomum villosum* Loureiro consist of similar chemical components, whereas *Amomum villosum* Loureiro var. *xanthioides* T.L. Wu et Senjen clearly differs from the above two types, and thus a comparison of pharmacological and clinical efficacies therebetween needs to be conducted.

Fatty liver is considered to be a pre-stage or symptom of metabolic diseases such as obesity, diabetes, and the like, and fatty liver development is described by a two-hit working model. Hepatic steatosis represents the first hit, which makes the liver vulnerable to second hits caused by liver damage, such as inflammation, saturated fatty acids, and the like. Recent studies have demonstrated that ER stress is an important factor as a second hit in this process, using ER stress markers such as GRP78, CHOP, and XBP-1, and thus it has become more important to study a method of reducing ER stress to prevent the aggravation of fatty liver.

Therefore, in the present study, to verify the efficacy of an *Amomum villosum* extract on preventing or alleviating fatty liver, HepG2 cells and a C57BL/6 mouse model, in which endoplasmic reticulum (ER) stress was induced by tunicamycin, which is a chemical inducer of fatty liver, were used to confirm the efficacy of the *Amomum villosum* extract *in vitro* and *in vivo,* thus completing the present invention.

Meanwhile, according to the related art, Korean Registered Patent Publication No. 10-1431610 discloses a pharmaceutical composition for preventing or treating obesity, dyslipidemia, fatty liver, or diabetes, which includes extracts from *Prunus mume Sieb. et Zucc.* And *Lithospermum erythrorhizom Sieb. et Zucc.* as active ingredients. Korean Registered Patent Publication No. 10-1206543 discloses that a composition for preventing or treating fatty liver including a *Castanea crenata* shell extract is effective.

However, the aforementioned cited references disclose the confirmation of efficacy using natural substances different from *Amomum villosum,* and thus are distinguished from the present invention.

### [Disclosure]

### [Technical Problem]

Therefore, the present invention has been made in view of the above problems, and it is an object of the present invention to provide a composition for preventing, treating, or alleviating fatty liver, which includes an *Amomum villosum* extract as an active ingredient.

It is an object of the present invention to provide a composition for preventing, treating, or alleviating fatty liver, which includes an *Amomum villosum* extract as an active ingredient, wherein the inhibitory efficacy of the *Amomum villosum* extract on developing ER-stress induced fatty liver and the mechanism therefor were confirmed *in vitro* and *in vivo* through inhibition of the expression of ER stress-related GRP78 and CHOP genes and the inhibition of fat synthesis-related SREBP-1 expression, upon administration of tunicamycin, which is an ER stress inducer, from which the effect of the *Amomum villosum* extract on preventing fatty liver was confirmed.

### [Technical Solution]

In accordance with the present invention, the above and other objects can be accomplished by the provision of a composition for preventing, treating, or alleviating fatty liver, which includes an *Amomum villosum* extract.

In the present invention, the *Amomum villosum* extract is included in a dose of 100 mg/kg to 500 mg/kg based on oral administration to a mouse.

In the present invention, the *Amomum villosum* extract is obtained through extraction with water.

In the present invention, the *Amomum villosum* extract inhibits fat production induced by tunicamycin in HepG2 cells and mice, and in the mice, inhibits protein and mRNA expression of the ER stress-related genes GRP78 and CHOP and inhibits protein and mRNA expression of the fatty liver synthesis-related genes, acetyl CoA carboxylase (ACC), fatty acid synthase (FAS), and sterol regulatory element binding protein (SREBP)-1.

In the present invention, the *Amomum villosum* extract inhibits ER stress-induced fatty liver development.

It is another object of the present invention to provide a health functional food composition for preventing or alleviating fatty liver and a liver disease, which includes an *Amomum villosum* extract as an active ingredient.

### [Advantageous effects]

According to an experimental example of the present invention, the efficacy of an *Amomum villosum* extract on inhibiting intrahepatic fat accumulation was examined *in vitro* and *in vivo* to confirm the efficacy of the *Amomum villosum* extract on preventing, treating, or alleviating fatty liver.

According to the experimental example of the present invention, as a result of treatment with an *Amomum villosum* extract, followed by treatment with tunicamycin, which is an ER stress inducer, it was confirmed that, in HepG2 cells and C57BL/6 mice, an increase in TG content was significantly inhibited by tunicamycin. In addition, the expression of ER stress-related genes, GRP78 and CHOP and fat synthesis-related SREBP-1 expression were inhibited.

From these results, it was verified that the *Amomum villosum* extract had an effect against fatty liver development due to ER stress and fat synthesis, which were induced by tunicamycin. Therefore, the inventors of the present invention developed a composition having an effect of preventing, treating, or alleviating fatty liver including an *Amomum villosum* extract, thus completing the present invention.

### [Description of Drawings]

FIG. 1 is a graph showing intracellular toxicity according to the concentration of an *Amomum villosum* extract through an experimental example of the present invention.
FIG. 2 is a graph showing the inhibition of intracellular TG production according to the concentration of an *Amomum villosum* extract through an experimental example of the present invention.
FIG. 3 is a graph showing the inhibition of TG production in animals according to the concentration of an *Amomum villosum* extract through an experimental example of the present invention.
FIG. 4 is a graph showing the effect of an *Amomum villosum* extract on tunicamycin-induced ER stress-related gene expression through an experimental example of the present invention.
FIG. 5 is a graph showing the effect of an *Amomum villosum* extract on tunicamycin-induced fat synthesis-related gene expression through an experimental example of the present invention.

### [Best Mode]

The terms or words used in the present specification and claims should not be construed as being limited to ordinary or dictionary meanings, but should be construed as having meanings and concepts consistent with the spirit of the present invention based on a principle that an inventor can appropriately define concepts of terms to explain the invention of the inventor in the best way.

Thus, details described in examples, reference examples, and drawings of the present specification are merely the most exemplary embodiments of the present invention and do not represent all technical spirits of the present invention, and thus it should be understood that various equivalents and modifications that may replace these embodiments can be made at the filing time of the present application.

### Experimental Example 1. Evaluation of Efficacy of Amomum villosum Extract on Inhibiting Fatty Liver Development

### 1) Reagent

*Amomum villosum,* which is a medicinal herb, was purchased from CoreSciences (Seoul, Korea). Tunicamycin was purchased from Sigma-Aldrich (St. Louis, MO, U.S.A.), and anti-glucose regulated protein 78 (GRP78), homolog protein (CHOP), and anti-X-box-binding protein-1 (XBP-1) were purchased from Santa Cruz Biotechnology (Santa Cruz, CA, U.S.A.). EZ-cytoxy, which is a cell viability assay reagent, was purchased from Sigma-Aldrich.

### 2) Amomum villosum Extraction Process

This process is to extract *Amomum villosum* with water to obtain an extract. Depending on the design conditions, different solvents may be used, and the extraction temperature, extraction time, amount of solvent, residual ingredient treatment method, and the like may be designed differently according to the type of extraction solvent. As the extraction solvent, various solvents may also be used, and extractable solvents include water, ethanol, methanol, fatty oil, glycerin, horse oil, ethyl acetate, acetone, butanol, isopropanol, and the like. Preferably, in the present invention, *Amomum villosum* was extracted with water.

### 3) Cell Viability Test

To evaluate efficacy for liver disease, human hepatocellular carcinoma cell line HepG2 was purchased from American Type Culture Collection (ATCC) (Manassas, VA, U.S.A.). HepG2 cells were cultured in Dulbecco's minimum Eagle's essential medium (DMEM) supplemented with 10% heat-inactivated fetal bovine serum (FBS) and 1% antibiotics.

EZ-cytoxy was used as a cell viability assay reagent. Formazan, produced using tetrazolium salt, was measured by absorbance. To evaluate the cytotoxicity of the *Amomum villosum* extract, the cytotoxicity of the *Amomum villosum* extract was examined according to various concentrations, i.e., 100 µg/ml, 200 µg/ml, 500 µg/ml, or 1,000 µg/ml, using EZ-cytoxy, which is a HepG2 cell viability assay reagent.

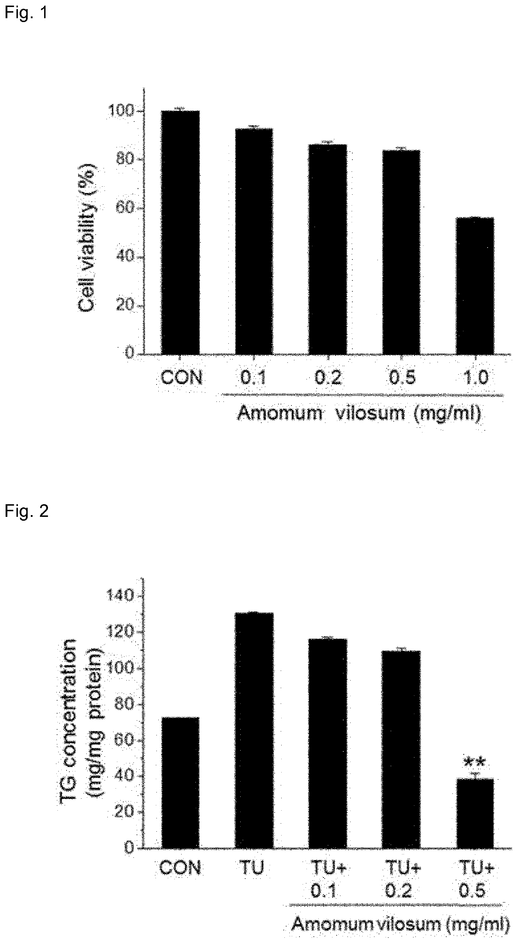

FIG. 1 is a graph showing intracellular toxicity according to the concentration of an *Amomum villosum* extract through an experimental example of the present invention.

As a result, 50% to 60% cell viability was confirmed at a concentration of 1,000 µg/ml, and significant cytotoxicity was not shown at a concentration of 100 µg/ml to 500 µg/ml. From the results, it was confirmed that the *Amomum villosum* extract had toxicity at a high concentration, and thus in the present invention, a concentration of 100 µg/ml to 500 µg/ml was used. In the present invention, the concentration of 1,000 µg/ml or more was excluded from the experiment due to high cytotoxicity, and three experimental groups administered an *Amomum villosum* extract at a concentration of 100 µg/ml, 200 µg/ml, or 500 µg/ml were used.

### 4) Evaluation of Efficacy of Amomum villosum Extract on Intracellular TG Inhibition

Human hepatocellular carcinoma cell line HepG2 was selected as hepatocytes to evaluate the efficacy of an *Amomum villosum* extract on intracellular TG inhibition. HepG2 cells were cultured in Dulbecco's minimum Eagle's essential medium (DMEM) supplemented with 10% heat-inactivated fetal bovine serum (FBS) and 1% antibiotics.

To investigate the effect of the *Amomum villosum* extract on inhibiting triglycerides (TG) increased by ER stress, the HepG2 cells treated with the *Amomum villosum* extract according to various concentrations (100 µg/ml, 200 µg/ml, or 500 µg/ml) and a control were cultured for 16 hours. After three hours, each group was treated with tunicamycin (2 µg/ml) and continued to be cultured for 24 hours.

After culturing, to measure TG content, the HepG2 cells were homogenized in a chloroform-methanol-H₂O solution (8:4:3, v/v/v). The cells were incubated at room temperature for 1 hour and centrifuged (3,000 rpm, 10 min). A supernatant was removed therefrom, and then a precipitate was dried for 24 hours. After dissolving the dried precipitate in ethanol, hepatic triglycerides (TG) were examined using a TG kit (AM 157S-K and AM 202-K, Asan Pharmaceutical, Korea) and normalized to a protein concentration.

FIG. 2 is a graph showing the inhibition of intracellular TG production according to the concentration of an *Amomum villosum* extract through an experimental example of the present invention.

As a result, the content of triglycerides (TG) was significantly increased in the case of tunicamycin compared to a control. The increase in TG content induced by tunicamycin was inhibited in a concentration-dependent manner in the group pre-treated with the *Amomum villosum* extract. That is, as a result of TG assay in *vitro*, it was confirmed that the *Amomum villosum* extract suppressed the increased TG level induced by tunicamycin in the HepG2 cells in a concentration-dependent manner. These results showed that the *Amomum villosum* extract had an effect of reducing an intracellular TG level in the HepG2 cells.

### 5) Effect of Amomum villosum Extract on Hepatic TG Level in Mouse Model

To confirm the *in vitro* effect of the *Amomum villosum* extract on inhibiting ER stress-induced TG accumulation in an *in vivo* model, the *Amomum villosum* extract at various concentrations and then tunicamycin were administered to C57BL/6 mice, and the content of TG accumulated in the liver was measured.

8-week-old C57BL/6 mice having a body weight of 24 g to 26 g were purchased from Central Lab. Animal Inc. (Seoul, Korea). The mice were randomly divided into 5 groups (n=6): a non-administered group (control); a positive control (group treated with tunicamycin alone); a group administered tunicamycin and 100 mg/kg of the *Amomum villosum* extract; a group administered tunicamycin and 200 mg/kg of the *Amomum villosum* extract; and a group administered tunicamycin and 500 mg/kg of the *Amomum villosum* extract. To adapt each group to the environment for three days, each group was sufficiently fed a general compound feed and water in an animal rearing room.

The *Amomum villosum* extract was orally administered for 7 days. On day 7, tunicamycin (1 mg/kg body weight) was administered via intraperitoneal injection for 24 hours. Animal experiments were approved by the Wonkwang University Animal Experiment Ethics Committee, and the use and management of laboratory animals were in accordance with institutional guidelines.

Hepatic lipid was extracted from the liver according to the following procedures. Liver tissue was homogenized in a chloroform-methanol solution (2:1, v/v), incubated at room temperature for 1 hour, and centrifuged (3,000 rpm, 10 min). A supernatant was removed therefrom, and then a precipitate was dried for 24 hours. After dissolving the dried precipitate in ethanol, hepatic triglycerides (TG) were examined using a TG kit (AM 157S-K and AM 202-K, Asan Pharmaceutical, Korea) and normalized to a protein concentration.

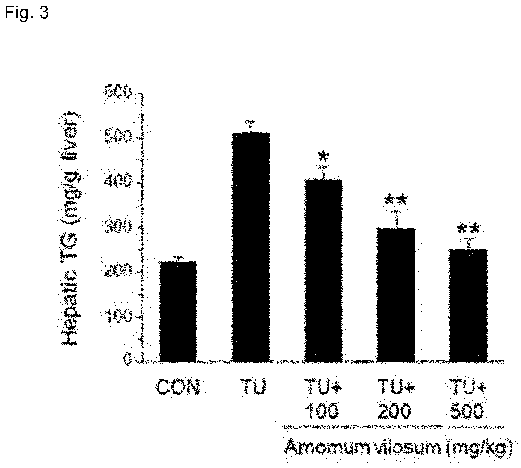

FIG. 3 is a graph showing the inhibition of TG production in animals according to the concentration of an *Amomum villosum* extract through an experimental example of the present invention.

As a result, the tunicamycin-administered group exhibited a two-fold or greater increase in hepatic TG content compared to the control, whereas the groups treated with the *Amomum villosum* extract inhibited tunicamycin-induced hepatic fat accumulation in a concentration-dependent manner. That is, as a result of conducting the in vivo experiment by pre-treating C57BL/6 mice with the *Amomum villosum* extract, injecting tunicamycin to each mouse for 24 hours, and then measuring hepatic TG levels, hepatic TG levels were remarkably increased after the injection of tunicamycin. However, the *Amomum villosum* extract reduced the increased TG level in a concentration-dependent manner. From these results, the effect of the *Amomum villosum* extract on inhibiting TG accumulation was also confirmed in the *in vivo* model.

### 6) Effect of Amomum villosum Extract on Expression of Tunicamycin-induced ER Stress-related Genes

In the present invention, to confirm whether the alleviation of fatty liver by the *Amomum villosum* extract is associated with a decrease in ER stress, protein and mRNA levels of the related genes GRP78 and CHOP, which are ER stress markers, were measured.

To investigate the inhibitory effect of the *Amomum villosum* extract on ER stress, the *Amomum villosum* extract was administered at various concentrations to C57BL/6 mice for 7 days. Subsequently, each mouse was administered tunicamycin, which is a pharmaceutical ER stress inducer, and the protein and mRNA levels of ER stress-related genes, GRP78 and CHOP in the liver were measured.

A reaction mixture containing a cDNA template, primers, and SYBR Green PCR Master MIX and a 7500 Fast RT-PCR system were used to perform real time PCR, and total RNA was isolated from laboratory mice using TRIzol (Invitrogen, Darmstadt, Germany).

In addition, proteins (40 µg per well) were separated on an 8% gel by sodium dodecyl sulfate-polyacrylamide gel electrophoresis (SDS-PAGE) and transferred to a polyvinylidene difluoride (PVDF) membrane. The blocked membrane was cultured with primary antibodies against 78-kDa glucose-regulated protein (GRP78;BiP) & C/EBP homologous protein (CHOP), which are ER stress markers. The proteins were examined using an enhanced chemiluminescence (ECL) Western blot detection kit (Amersham, Uppsala, Sweden).

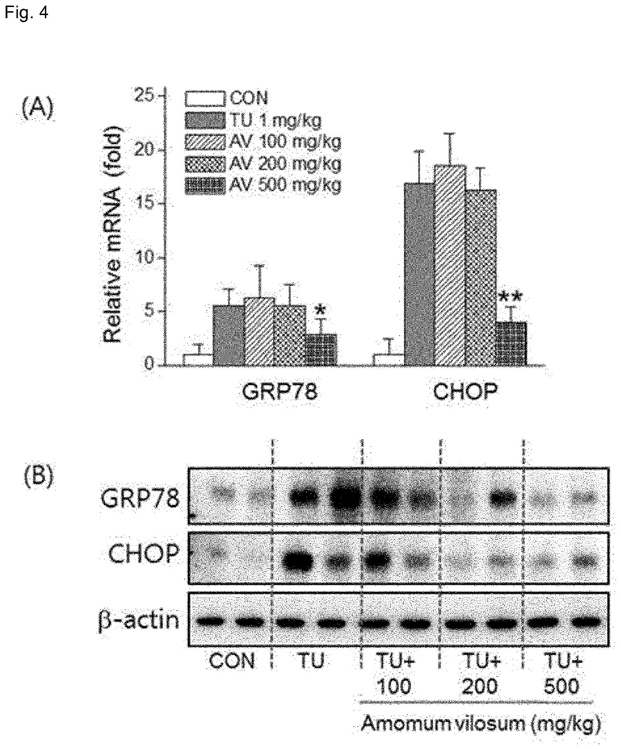

FIG. 4 is a graph showing the effect of an *Amomum villosum* extract on tunicamycin-induced ER stress-related gene expression through an experimental example of the present invention.

As a result, the expression of GRP78 protein and mRNA and CHOP protein and mRNA was significantly reduced in the liver in the groups administered tunicamycin after the *Amomum villosum* extract, compared to the positive control administered tunicamycin. From the results, it was confirmed that the *Amomum villosum* extract had inhibitory efficacy against the pharmaceutical ER stress inducer in hepatocytes.

### 7) Effect of Amomum villosum Extract on Expression of Tunicamycin-induced Fat Synthesis-related Genes

To discover the mechanism for the effect of the *Amomum villosum* extract on protecting the liver, the effect of the *Amomum villosum* extract on lipogenesis was examined.

That is, to confirm the mechanism for the effect of the *Amomum villosum* extract on inhibiting ER stress-induced TG, the *Amomum villosum* extract was administered at various concentrations to C57BL/6 mice for 7 days. Subsequently, each mouse was administered tunicamycin, which is an ER stress inducer to measure the expression levels of hepatic fat synthesis-related genes, acetyl CoA carboxylase (ACC), fatty acid synthase (FAS), and sterol regulatory element binding protein (SREBP)-1.

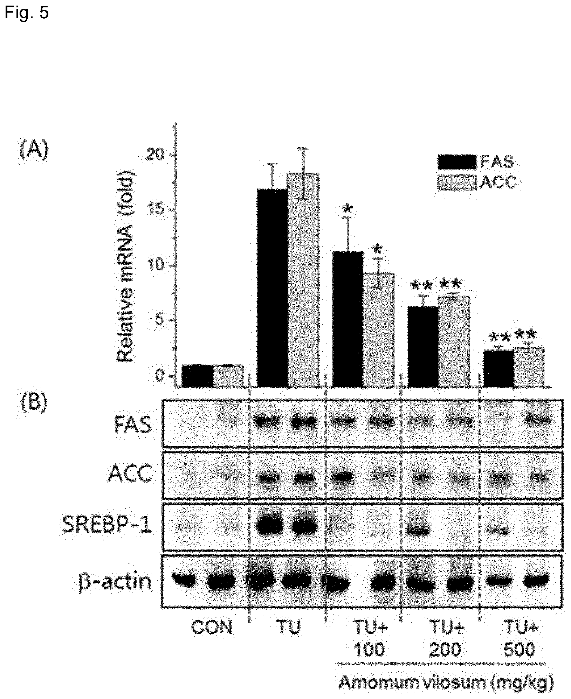

FIG. 5 is a graph showing the effect of an *Amomum villosum* extract on tunicamycin-induced fat synthesis-related gene expression through an experimental example of the present invention.

As a result, the levels of ACC mRNA and FAS mRNA, which are known as hepatic fat synthesis-inducing genes, were significantly reduced in the groups administered the *Amomum villosum* extract, compared to the tunicamycin-administered control. In addition, the expression of SREBP-1, which is known as a transcription factor for affecting the expression of ACC and FAS, was significantly reduced compared to the tunicamycin-administered control.

In the previous studies, it has been known that ER stress activates SREBP-1, which is a major fat-forming transcription factor, thereby increasing the expression of lipogenic genes such as ACC and FAS. This demonstrates that the *Amomum villosum* extract inhibits ER stress-promoted SREBP-1 expression, resulting in inhibition of the expression of lipogenic genes such as FAS and ACC, thereby alleviating fatty acid.

### 8) Statistical Processing

The data was expressed as mean±standard error of mean (S.E.M.). Statistically significant differences were measured by one-way ANOVA, followed by a Duncan's multiple-range test. In statistical analysis, p values less than or equal to 0.05 are considered significant.

### 9) Review

To confirm the efficacy of the *Amomum villosum* extract on preventing, treating, or alleviating fatty liver, the inhibitory efficacy thereof on TG accumulation was examined *in vitro* and *in vivo.*

First, the *Amomum villosum* extract did not exhibit toxicity in HepG2 cells up to the concentration of 500 µg/ml. Therefore, as a result of treating the cells with each concentration of the *Amomum villosum* extract, followed by treatment with tunicamycin, which is an ER stress inducer, it was confirmed that the *Amomum villosum* extract significantly inhibited the tunicamycin-induced increase in TG content in HepG2 cells and C57BL/6 mice.

In addition, the *Amomum villosum* extract inhibited the expression of ER stress-related genes GRP78 and CHOP and fat synthesis-related SREBP-1 expression. From the results, it was verified that the *Amomum villosum* extract had efficacy on fatty liver development due to ER stress and fat synthesis, which were induced by tunicamycin. Therefore, in the present invention, a composition for preventing, treating, or alleviating fatty liver, which includes an *Amomum villosum* extract, was developed.

## Claims

1. A health functional food composition for preventing or alleviating fatty liver development induced by endoplasmic reticulum (ER) stress, the health functional food composition comprising an *Amomum villosum* extract as an active ingredient,
wherein the *Amomum villosum* extract is obtained through extraction with water and included in a dose of 100 mg/kg to 500 mg/kg based on oral administration to a mouse, and
the *Amomum villosum* extract inhibits triglyceride production induced by tunicamycin in HepG2 cells and mice, and in the mice, inhibits protein and mRNA expression of ER stress-related genes GRP78 and CHOP, and protein and mRNA expression of fatty liver synthesis-related genes, acetyl CoA carboxylase (ACC), fatty acid synthase (FAS), and sterol regulatory element binding protein (SREBP)-1.
